# EUROPEAN PATENT SPECIFICATION

(11) **EP 2 135 629 B1**
(45) Date of publication and mention of the grant of the patent: **15.02.2012**
(21) Application number: 08381021.8
(22) Date of filing: 17.06.2008
(51) Int. Cl.: A61M 1/00, G05D 16/06

(54) **Measurement device for vacuum regulator**
Messvorrichtung für Vakuumregler
Dispositif de mesure pour régulation sous vide

(43) Date of publication of application: 23.12.2009
(73) Proprietor: Hersill, S.L., 28935 Mostoles Madrid (ES)
(72) Inventor: Martinez Jordan, Miguel Oscar, 28935 MOSTOLES (ES); Martinez Jordan, Carlos, 28935 MOSTOLES (ES)
(74) Representative: Carpintero Lopez, Francisco

(56) References cited:
- WO-A-00/76563
- DE-U1- 8 010 779
- GB-A- 2 348 265
- US-A- 4 961 726
- US-A1- 2007 219 537

## Description

### OBJECT OF THE INVENTION

This invention refers to a measurement device for a vacuum regulator. The invention is particularly suitable for controlling vacuum in a circuit applied to a patient for medical purposes.

A vacuum regulator is a device of the type comprising an entry of a vacuum source, an outlet of a vacuum circuit, generally on the patient's side and means for regulating the pressure supplied to the vacuum circuit. The measurement device comprises an external vessel, an elastic element situated internally to the external vessel and which may be displaced in a linear manner along said vessel, an impermeable elastic membrane which is displaced solid to the elastic element and which separates the vacuum source requiring measurement from the atmosphere, and, finally, means for indicating the pressure of the vacuum circuit in correspondence with the linear movement of the elastic element.

The invention is characterised by the characterizing part of claim 1.

### BACKGROUND TO THE INVENTION

The creation of a vacuum in a circuit is widely used in hospitals, for example, to extract fluids from body cavities.

Vacuum regulating devices are already known, especially those used in the hospital environment, the object of which is to control the vacuum generated in the circuit in contact with the patient. These devices permit the control and maintenance of a negative pressure with respect to the atmospheric pressure in a circuit associated with a patient, without the need to interrupt any of the patient's connections.

These vacuum regulating devices require a measurement device which permits control, normally visual, of the pressure which is being administered to the patient.

Measurement devices are known, for example, vacuum gauges which are provided with a needle which rotates in respect of a perpendicular axis and which indicate the pressure supplied on a numerical scale. Nevertheless, the vacuum gauges have the basic disadvantage of being delicate devices, and which are difficult or impossible to sterilise, in addition to requiring tools for their calibration.

This type of device also needs to be sealed, so that the part in contact with the vacuum source to be measured does not leak, which could contribute to a pressure leak in the circuit.

There are other types of indicator in which indication is achieved by linear displacement along a graduated scale of a device moved directly by the pressure of the circuit. In this type of device the seal may be achieved by means of, for example, a ring seal which seals the part of the device in contact with the pressure requiring measurement. Nevertheless, this type of sealed closure produces friction in the linear displacement which may distort the measurement obtained, that is, it is difficult to comply with the restrictions of accuracy or uncertainty required for vacuum regulators for medical use. Another.known solution is that of substituting the ring seal with a guided cover used as slide.

Included in this last type of device there are some which achieve linear displacement as they consist of a spring which moves a piston which is surrounded by an impermeable membrane, thus obtaining the aforementioned seal. However, this type of device has the disadvantage that with the linear displacement of the piston, the membrane rolls between the aforementioned piston and the external body so that this rolling movement of the membrane causes friction which affects the accuracy of the measurement. Furthermore, the use of the piston requires additional space of the external body in respect of which the spring is displaced for at least half the carriage of the aforementioned piston.

It also known from the state of the art a measurement device for vacuum regulator as the one disclosed in DE 1080779 U1 which discloses a vacuum regulator having an external vessel, a bellows and a spring housed inside the bellows.

The present invention resolves the above mentioned technical problems by means of a sterilisable device which cancels friction in the linear displacement of the measurement element and which restricts the additional space to that of measurement required for the displacement of any of its elements to the height of the spring in such a way that almost all the linear displacement is useful along its entire path thus achieving a compact device, easy to read with adequate accuracy and uncertainties and with the possibility of adjustment of the scale from zero.

### DESCRIPTION OF THE INVENTION

This invention refers to a measurement device for a vacuum regulator.

The vacuum regulator is of the type which consists of at least one external body comprising an entry into a vacuum source, an outlet to a vacuum circuit which will normally be in contact with the patient, the force of which is used to extract bodily fluids or surgical serum of said patient, and means for regulating the pressure supplied to the outlet of the vacuum circuit.

The measurement device is of the type which comprises:
- An external vessel
- An elastic element situated internally to the external vessel and which may be linearly displaced along the same. This displacement occurs through the action of the pressure which, if it is negative, compresses the elastic element, and if it is positive, expands it.
- An elastic impermeable membrane which is displaced solidly to the elastic element and which separates the vacuum circuit of the patient, the magnitude of which requires measurement of the atmospheric pressure. The elastic membrane prevents leaks to the exterior perfectly insulating the pressure of the vacuum circuit; and finally,
- Indication means which permit the reading of the magnitude of the vacuum circuit pressure in correspondence with the linear movement of the elastic element.

The invention is characterised by the fact that the elastic element is embedded in the interior of the impermeable elastic membrane in such a way that one of the faces of the elastic membrane is in contact with the vacuum circuit requiring measurement.

The external vessel may be totally or partially transparent so that it is possible to visualise the elastic element from the outside.

Therefore, the linear displacement of the device essentially involves two elements, that is, the elastic element and the membrane into which it is embedded.

The embedding of the elastic element in the impermeable membrane interior may be achieved by the technique of applying vacuum in the exterior of the membrane in order to expand it until it is adapted to a part similar to the external vessel, subsequently it is introduced into the elastic element and finally the vacuum is removed so that the membrane is embedded thus covering the elastic element.

This type of measurement device may be installed in a vacuum regulator which is either continuous or intermittent, or which has both functions.

### DESCRIPTION OF THE DRAWINGS

The present descriptive report is supplemented by a series of drawings illustrative of a preferred embodiment but not however restricting the invention in any way.
Figure 1 shows a cross section diagram of an example of an embodiment of the measurement device which is the object of the invention arranged on a vacuum regulator of the intermittent type.
Figure 2 shows a perspective diagram of an example of an embodiment of the external vessel of the measurement device.
Figure 3 shows a cross section of the main body of the intermittence mechanism of the example of an embodiment corresponding to figure 1 which represents a first situation in which the intermittence mechanism is in an on position, that is, there is a vacuum flow between the entry and outlet thereof.
Figure 4 is a cross section of the main body of the intermittence mechanism of the example of an embodiment corresponding to figure 1 in which it represents a case in which the system is in a transition situation between on and off.
Figure 5 is a cross section of the main body of the intermittence mechanism of the example of an embodiment corresponding to figure 1 which represents a situation in which there is no vacuum flow between the entry and the outlet thereof and thus it is in the off position.
Figure 6 shows a diagram of a medical aspiration system using a vacuum regulator.

### PREFERRED EMBODIMENT OF THE INVENTION

The vacuum regulator shown in the figures is of the intermittent type, although the measurement device which is the object of the invention could be installed in any type of vacuum regulator, for example continuous.

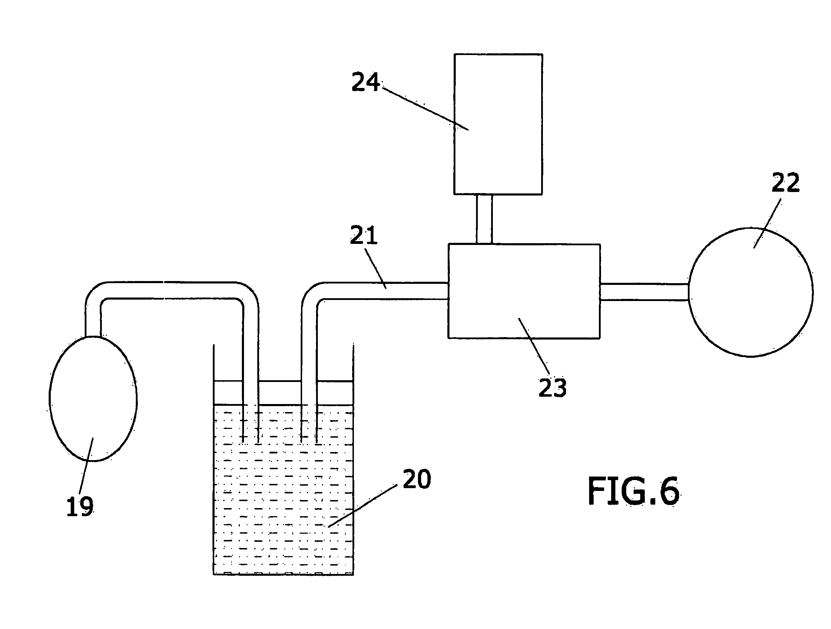

Figure 6 shows a diagram of an example of an embodiment of a medical aspiration system which makes use of a vacuum regulator. The system is situated in connection with a patient (19) who is connected to a deposit (20) which collects the patient's (19 liquids to which in turn a vacuum circuit (21) is joined, which communicates with a vacuum source (22) with a vacuum regulator (23) existing between both (21, 22) which includes a measurement device (24).

The vacuum regulator is the type which consists of at least an external body (1) which comprises an entry (2) from a vacuum source and an outlet (3) to a vacuum circuit which will normally be in contact with the patient and through which the fluids which are extracted from that patient circulate.

The measurement device is of the type which in turn comprises an external vessel (15). An elastic element (16) which in the embodiment shown corresponds to a compressed helical spring situated internally to the external vessel (15) and which may be displaced in a linear manner along the same. An impermeable elastic membrane (17) embedded in the spring (16), one of whose faces is in contact with the patient's vacuum circuit the magnitude of which is to be measured.

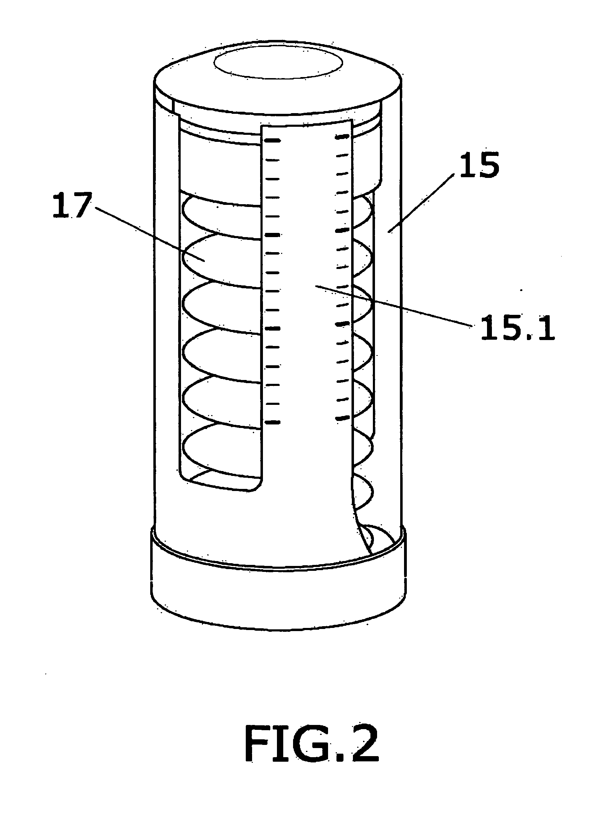

The means of indication of the pressure reading, see figure 2, may correspond to a scale (15.1) which may be screen printed on the actual external vessel (15) so that the upper part of the elastic element (16) when it is displaced, marks the pressure reading on the scale (15.1)

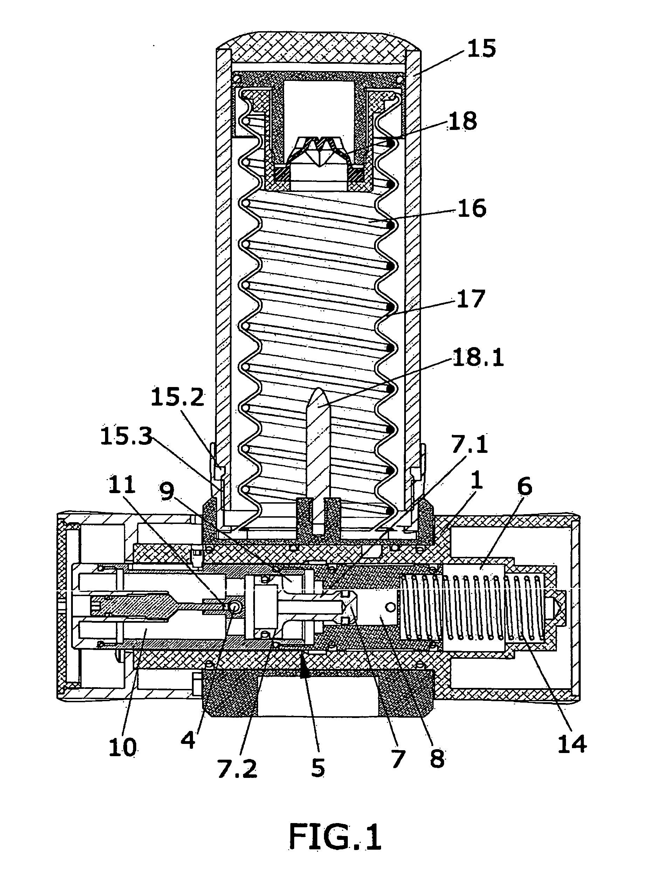

In the embodiment shown in figure 1 the measurement device also comprises an additional device which enables the pressure in the interior of the membrane (17) to be limited (the patient's vacuum circuit) both in the maximum limit of negative pressure (maximum vacuum, and positive pressure. This device comprises a valve (18) made from elastic material provided with a geometry which is such that while there is a negative pressure (vacuum) in the internal chamber of the membrane (17), the valve (18) remains closed, whereas if there is a positive pressure the valve (18) will open to communicate the internal chamber with the atmosphere, and in this way it protects the patient against a positive pressure. This valve (18) has a second function which is that of protecting the patient against a vacuum greater than the maximum of the scale of the measurement device, this is produced when the spring (16) is compressed to the point in which the rod (18.1) touches the valve (18) at which moment valve (18) is forced to open, communicating the vacuum chamber with the atmosphere.

The external vessel (15) of the measurement device is joined to the external body (1) of the vacuum regulator by means of a screw thread (15.3) which is provided with a clearance (15.2) which permits correct adjustment of the pressure scale.

Figure 1 shows an example of an embodiment of an intermittent vacuum regulator. It is provided with an external body (1), an entry (2), fed by a vacuum source and an outlet (3) to a vacuum circuit on the patient's side.

The intermittence of the vacuum regulator shown in the figures is achieved because there is at least one connection (4) between the entry (2) and the outlet (3). This connection (4) comprises means which permit its variation between at least one turned on position in which there is a communication between the entry (2) and the outlet (3) so that the circuit on the patient's side receives vacuum from the aforementioned vacuum source. It may also be provided with an off position in which communication between the entry (2) and outlet (3) is prevented, thus vacuum or suction is not transmitted to the patient's circuit.

The intermittence mechanism of the embodiment shown in the figures is characterised in that it comprises the following elements:
- an intermediate body (5) which may be displaced according to a longitudinal axis in the interior of the external body (1). This intermediate body (5) includes an associated moveable connection (4), and in this way the axial displacement of the intermediate body (5) permits the connection (4) to communicate the entry (2) and the outlet (3), that is, turning it on, or instead it prevents it. The relative displacement of the intermediate body (5) is achieved according to the pressure obtained in an end chamber (6) between the external body (1) and the transversal end of the intermediate body (5).
- an internal piston (7), which is axially displaceable in respect of the intermediate body (5) in such a way that its displacement enables or prevents entry of vacuum in the end chamber (6) and therefore the displacement of the internal body (5).

That is, the greater or lesser pressure of the end chamber (6) causes the displacement of the intermediate body (5) which displaces the moveable connection (4) between the entry (2) and the outlet (3), preventing or permitting vacuum flow from the entry (2) to the outlet (3). Regulation of this pressure in the end chamber (6) is achieved by displacing the internal piston (7) which passes from a position in which it permits the vacuum flow to the end chamber (6) to another in which it is prevented.

In addition, the transversal end of the intermediate body (5) is subject to the force of an elastic element which tends to expand the end chamber. In the embodiment shown the elastic element materialises in a compressed helical spring (14).

In the embodiment shown in the figures, the piston (7) comprises sections (7.1, 7,2) with different diameter. The internal piston (7) defines three chambers, a first chamber (8) between the end of the smaller section (7.1) of the piston (7), the intermediate body (5) and its transversal end, a second chamber (9) defined between the piston (7) and the intermediate body (5) between the points of contact in both sections (7.1, 7.2) and finally a third chamber (10) between the intermediate body (5) and the end of the larger section (7.2) of the internal piston (7).

The displacement of the piston (7) occurs through the difference in pressure between the first (8) and third (10) chambers also taking into account the difference in section (7.1, 7.2) between each end of the piston (7).

In some vacuum regulator devices, the displacement of the elements which ensures communication between the entry (2) and the outlet (3) is made for example by magnetic means. However, in the embodiment shown this is achieved by means of a conduct (11) between the displaceable connection (4) (which links the entry (2) to the outlet (3) and the chamber (10), with less flow than the connection (4) between the entry (2) and the outlet (3). In this way when vacuum circulates in the connection (4) between the entry (2) and the outlet (3), part of it is transferred to the third chamber (10) which gradually lowers its pressure.

There is also a connection (12) between the end chamber (6) and, or either the first chamber (8) or either the second chamber (9) based on the displacement of the piston (7).

Finally, there is also a vacuum connection (13) between the entry (2) and the first chamber (8).

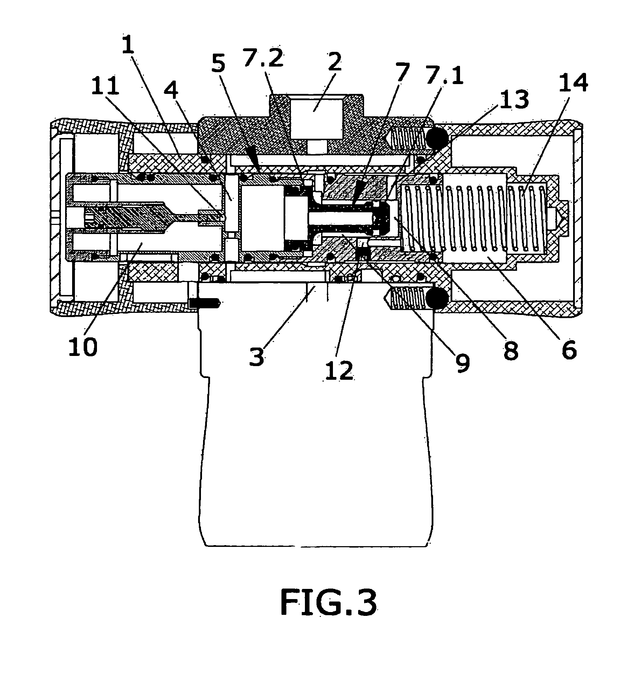

Figure 3 shows a first situation with the intermittence mechanism in the on position, that is, there is a vacuum flow between the entry (2) and the outlet (3) through the corresponding moveable connection (4).

The vacuum flow circulates through the moevable connection (4) and through the connection between the entry (2) and the first chamber (8). Therefore, there is a vacuum zone and the remainder is situated at atmospheric pressure. In turn there is leakage through the lesser flow conduct (11) to the third chamber (10). In this way through the leakage from the lesser flow conduct (11) a vacuum or depression is generated in the interior of the third chamber (10) in a gradual manner.

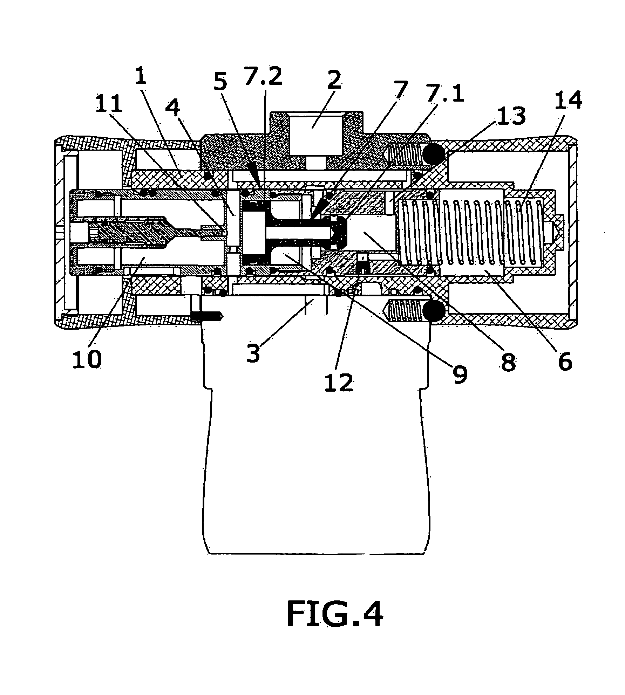

At some point the vacuum generated in the third chamber (10) and due to the difference in section (7.1, 7.2) of the internal piston (7) will be greater than that existing in the first chamber (8) in such a way that it will produce a displacement of the piston (7) with respect to the intermediate body (5). This situation is represented in figure 4.

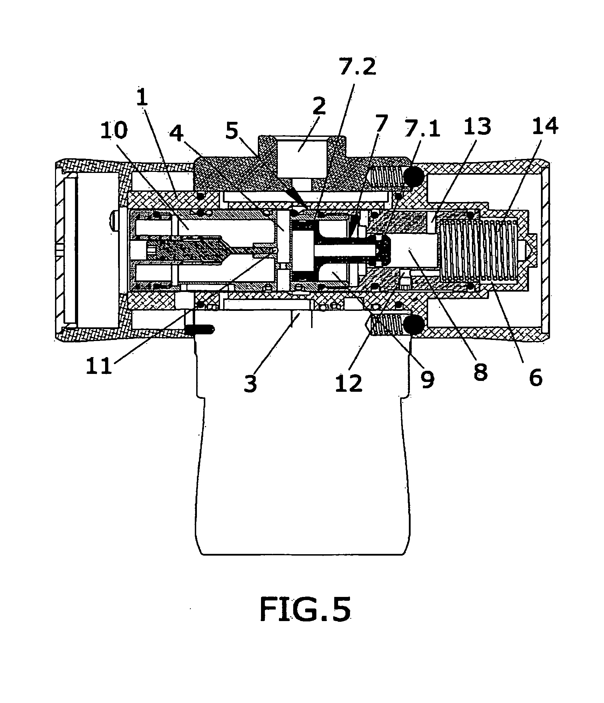

When the internal piston (7) is displaced, the connection opens (12) between the first chamber (8) and the end chamber (6) so that a vacuum is generated in the aforementioned chamber (6). This causes the intermediate body (5) to retract so that it transfers the moveable connection (4) between the entry (2) and the outlet (3) and thus there is no vacuum flow between the entry (2) and the outlet (3), see figure 5.

As the moveable connection (4) enters into contact with the atmospheric pressure, there is slow leakage through the conduct (11) to the third chamber (10) in a way in which the process is inverted. That is, through the conduct (11) between the moveable connection (4) which is now at atmospheric pressure, atmospheric pressure progressively passes to the third chamber (10) in such a way that it will reach a point where the chamber (10) is at atmospheric pressure, whereas the first chamber (8) is in vacuum, and therefore the piston (7) will once more displace towards the first chamber (8) closing the connection between this chamber (8) and the end chamber(6) which, due to displacement of the piston (7) joins the second chamber (9) and the end chamber (6), and thus this (6)receives atmospheric pressure and therefore the force of the spring (14)displaces the intermediate body (5)in such a way that once more the connection (4) between the entry (2) and the outlet (3) permits the flow of vacuum between both elements (2,3) with all the elements returning to their position as shown in figure 3.

In the preferred embodiment the intermediate body (5) is formed by the connection of two bodies.

The vacuum regulator device also comprises selector controls so that by turning these it is possible to vary between the intermittence position such as that which has been explained, or either with continuous flow or either turned off. The device also comprises a pressure regulator which permits the working pressure to be selected.

## Claims

1. A medical vacuum regulator, comprising a measurement device comprising:
an external vessel (15),
an elastic element (16) arranged internally to the external vessel (15) and which may be linearly displaced along the external vessel (15), said elastic element (16) consisting of a helical spring configured to be compressed when negative pressure is applied to the external vessel,
an impermeable membrane (17) which may be displaced solidly with the elastic element (16), and which is provided for separating a patient's vacuum circuit, the magnitude of which needs to be measured, from the atmospheric pressure,
means for indicating the magnitude of the pressure measurement in correspondence with the linear displacement of the elastic element (16),
**characterised in that**,
the impermeable membrane (17) is made of an elastic material, and wherein the helical spring (16) is embedded in the internal face of the impermeable membrane (17) in such a way that one of the internal face of the elastic membrane (17) is arranged to be in contact with a vacuum circuit to be measured,
and wherein it further comprises one way valve (18), arranged for limiting the pressure in the interior of the impermeable membrane (17), said one way valve (18) provided with means for its opening when the pressure descends below the pre-established value and leaks into the atmosphere in the event of positive pressure.

2. Vacuum regulator, according to claim 1, **characterised in that** the external vessel (15) is transparent, and the indication means are indicated thereon.

3. Vacuum regulator, according to claim 2, **characterised in that** the means of indication consist of at least one scale (15.1) situated on the wall of the external vessel (15).

4. Vacuum regulator, according to claim 4, **characterised in that** the means of opening when the pressure descends comprise an actuator (18.1)which enters into contact with the valve (18) when the descent of the elastic element (16) reaches a predetermined value, thus opening the same (18).

5. Medical aspiration system comprising a vacuum regulatory according to any of the preceding claims wherein the measurement device is installed in the vacuum regulator for measuring pressure therein, wherein the vacuum regulator comprises an entry (2) to a vacuum inlet and an outlet (3) to a vacuum circuit on a patient's side and means of regulating the pressure supplied to the outlet to a vacuum circuit.

6. System according to claim 5, **characterised in that** the vacuum regulator further comprises an intermittence mechanism which includes at least one moveable connection (4) between the on position which communicates the vacuum entry (2) and outlet (3) and an off position in which there is no communication between entry (2) and outlet (3), additionally including:
an intermediate body (5), moveable according to a longitudinal axis in the interior of the external body (1) and which comprises an associated moveable connection (4) such that the axial displacement of the intermediate body (5) permits the connection (4)in order to either permit or prevent communication between the entry (2) and the outlet (3); and which defines an end chamber (6) between the external body (1) and the transversal end of the intermediate body (5) of displacement of the intermediate body (5) based on the pressure reached by said chamber (6).
a piston (7)internal to the intermediate body (5) and which is axially moveable in respect of that intermediate body (5) in such a way that its displacement enables or prevents entry of vacuum in the end chamber (6) and therefore the displacement of the internal body (5).

7. System according to claim 6, **characterised in that** the end chamber (6) is subject to an elastic force.

8. System according to claim 7, **characterised in that** the elastic force is exercised by a spring (14).

9. System according to claim 6, **characterised in that** the piston (7) comprises end sections (7.1, 7.2) with different diameters and which defines three chambers, a first chamber (8) between the end of the smaller section (7.1) of the piston(7), the intermediate body (5) and its transversal end, a second chamber (9) defined between the piston (7) and the intermediate body (5) between the points of contact in both sections (7.1, 7.2) and finally a third chamber (10) between the intermediate body (5) and the end of the larger section (7.2) of the internal piston(7).

10. System according to claim 9, **characterised in that** it comprises a connection (11) between the moveable connection (4) and the third chamber (10) with less flow than the connection (4) between the entry (2) and the outlet (3).

11. System according to claim 9, **characterised in that** it includes a connection (12) between the end chamber (6) and, or either the first chamber (8) or either the second chamber (9) based on the displacement of the piston (7).

12. System according to claim 9, **characterised in that** it comprises a connection (13) between the entry (2) and the first chamber (8).

13. System according to claim 6, **characterised in that** the intermediate body (5) comprises respective bodies.

## Patentansprüche

1. Medizinischer Vakuumregler mit einer Messvorrichtung, enthaltend:
einen Außenbehälter (15),
ein elastisches Element (16), das im Inneren des Außenbehälters (15) angeordnet ist und das entlang des Außenbehälters (15) linear verschoben werden kann, wobei das elastische Element (16) aus einer Schraubenfeder besteht, die dazu eingerichtet ist, zusammengedrückt zu werden, wenn ein Unterdruck auf den Außenbehälter wirkt,
eine undurchlässige Membran (17), die fest mit dem elastischen Element (16) verschoben werden kann und dazu vorgesehen ist, den Vakuumkreislauf eines Patienten, dessen Größe zu messen ist, von dem atmosphärischen Druck zu trennen, und
Einrichtungen zum Anzeigen der Größe der Druckmessung in Korrespondenz mit der linearen Verschiebung des elastischen Elementes (16),
**dadurch gekennzeichnet, dass**
die undurchlässige Membran (17) aus einem elastischen Material besteht und die Schraubenfeder (16) in die Innenfläche der undurchlässigen Membran (17) derart eingebettet ist, dass eine der Innenflächen der elastischen Membran (17) so angeordnet ist, dass sie mit dem zu messenden Vakuumkreislauf in Kontakt ist,
und sie weiterhin ein Rückschlagventil (18) enthält, das dazu eingerichtet ist, den Druck im Inneren der undurchlässigen Membran (17) zu begrenzen, wobei das Rückschlagventil (18) mit Einrichtungen zum Öffnen desselben ausgestattet ist, wenn der Druck unter den vorab eingerichteten Druck abfällt und für den Fall eines Überdrucks in die Atmosphäre austritt.

2. Vakuumregler nach Anspruch 1, **dadurch gekennzeichnet, dass** derAußenbehälter (15) transparent ist und die Anzeigeeinrichtungen darauf markiert sind.

3. Vakuumregler nach Anspruch 2, **dadurch gekennzeichnet, dass** die Anzeigeeinrichtungen aus wenigstens einer Skala (15.1) bestehen, die sich auf der Wand des Außenbehälters (15) befindet.

4. Vakuum regler nach Anspruch 4, **dadurch gekennzeichnet, dass** die Öffnungseinrichtungen, wenn der Druck abfällt, ein Stellglied (18.1) enthalten, das in einen Kontakt mit dem Ventil (18) eintritt, wenn der Abfall des elastischen Elementes (16) einen vorbestimmten Wert erreicht, wodurch sich dieses (18) öffnet.

5. Medizinisches Ansaugsystem, enthaltend einen Vakuum regler nach einem der beiden vorhergehenden Ansprüche 1 bis 2, bei dem die Messvorrichtung in dem Vakuum regler zum Messen des Drucks in diesem angeordnet ist, wobei der Vakuumregler einen Einlass (2) zu einem Vakuumeinlass und einen Auslass (3) zu einem Vakuumkreislauf auf der Seite eines Patienten sowie Einrichtungen zum Regeln des Drucks enthält, der dem Auslass zu einem Vakuumkreislauf zugeführt wird.

6. System nach Anspruch 5, **dadurch gekennzeichnet, dass** der Vakuumregler weiterhin einen Unterbrechungsmechanismus enthält, der über wenigstens eine bewegliche Verbindung (4) zwischen der Ein-Stellung, die den Vakuumeinlass (2) und den Auslass (3) verbindet, und einer Aus-Stellung verfügt, in der keine Verbindung zwischen dem Einlass (2) und dem Auslass (3) besteht, zusätzlich enthaltend:
einen Zwischenkörper (5), der in Übereinstimmung mit einer Längsachse in dem Inneren des externen Körpers (1) beweglich ist und eine zugehörige bewegliche Verbindung (4) derart enthält, dass die axiale Verschiebung des Zwischenkörpers (5) die Verbindung (4) ermöglicht, um die Verbindung zwischen dem Einlass (2) und dem Auslass (3) entweder zuzulassen oder zu verhindern; und der eine Endkammer (6) zwischen dem externen Körper (1) und dem querverlaufenden Ende des Zwischenkörpers (5) infolge der Verschiebung des Zwischenkörpers (5) auf der Basis des Drucks bildet, der in der Kammer (6) erreicht wird; und
einen Kolben (7) im Inneren des Zwischenkörpers (5), der im Bezug auf den Zwischenkörper (5) derart in Achsrichtung verschoben werden kann, dass dessen Verschiebung den Eintritt eines Unterdrucks in die Endkammer (6) und somit die Verschiebung des Zwischenkörpers (5) ermöglicht oder verhindert.

7. System nach Anspruch 6, **dadurch gekennzeichnet, dass** die Endkammer (6) einer elastischen Kraft ausgesetzt ist.

8. System nach Anspruch 7, **dadurch gekennzeichnet, dass** die elastische Kraft von einer Feder (14) ausgeübt wird.

9. System nach Anspruch 6, **dadurch gekennzeichnet, dass** der Kolben (7) Endabschnitte (7.1, 7.2) mit unterschiedlichen Durchmessern enthält und drei Kammern bildet, eine erste Kammer (8) zwischen dem Ende des kleineren Abschnittes (7.1) des Kolbens (7), dem Zwischenkörper (5) und dessen querverlaufendem Ende, eine zweite Kammer (9), die zwischen dem Kolben (7) und dem Zwischenkörper (5) zwischen den Berührungspunkten in beiden Abschnitten (7.1, 7.2) gebildet ist, und schließlich eine dritte Kammer (10) zwischen dem Zwischenkörper (5) und dem Ende des größeren Abschnittes (7.2) des Innenkolbens (7).

10. System nach Anspruch 9, **dadurch gekennzeichnet, dass** es eine Verbindung (11) zwischen der beweglichen Verbindung (4) und der dritten Kammer (10) mit einem geringeren Durchfluss als jenen der Verbindung (4) zwischen dem Einlass (2) und dem Auslass (3) enthält.

11. System nach Anspruch 9, **dadurch gekennzeichnet, dass** es eine Verbindung (12) zwischen der Endkammer (6) und/oder der ersten Kammer (8) und/oder der zweiten Kammer (9) auf der Basis der Verschiebung des Kolbens (7) enthält.

12. System nach Anspruch 9, **dadurch gekennzeichnet, dass** es eine Verbindung (13) zwischen dem Einlass (2) und der ersten Kammer (8) enthält.

13. System nach Anspruch 6, **dadurch gekennzeichnet, dass** der Zwischenkörper (5) entsprechende Körper enthält.

## Revendications

1. Régulateur de vide médical comportant un dispositif de mesure ayant :
- un récipient externe (15),
- un élément élastique (16) logé à l'intérieur du récipient externe (15) et mobile linéairement le long du récipient externe (15), cet élément élastique (16) étant constitué par un ressort hélicoïdal configuré pour être comprimé lorsqu'une pression négative est appliquée au récipient externe,
- une membrane imperméable (17) mobile solidairement avec l'élément élastique (16) et qui sépare le circuit de vide du patient dont il faut mesurer le niveau de vide par rapport à la pression atmosphérique,
- un moyen pour indiquer l'amplitude de la mesure de pression correspondant au déplacement linéaire de l'élément élastique (16),
**caractérisé en ce que**
- la membrane imperméable (17) est en une matière élastique et le ressort hélicoïdal (16) est intégré dans la face interne de la membrane imperméable (17) de façon que l'une des faces internes de la membrane élastique (17) soit en contact avec le circuit de vide mesuré, et
- il comporte en outre une soupape unidirectionnelle (18) destinée à limiter la pression à l'intérieur de la membrane imperméable (17), cette soupape unidirectionnelle (18) comportant un moyen pour s'ouvrir si la pression descend en dessous de la valeur prédéfinie et qui communique avec l'atmosphère si la pression est positive.

2. Régulateur de vide selon la revendication 1,
**caractérisé en ce que**
le récipient externe (15) est transparent et porte des indications.

3. Régulateur de vide selon la revendication 2,
**caractérisé en ce que**
les indications se composent d'au moins une échelle (15.1) sur la paroi du récipient externe (15).

4. Régulateur de vide selon la revendication 3,
**caractérisé en ce que**
le moyen d'ouverture lorsque la pression descend se compose d'un actionneur (18.1) qui vient en contact avec la soupape (18) lorsque l'élément élastique (16) descend jusqu'à une valeur prédéfinie, de manière à ouvrir la soupape (18).

5. Système d'aspiration médical comportant un régulateur de vide selon l'une quelconque des revendications 1 et 2,
**caractérisé en ce que**
- le dispositif de mesure est installé dans le régulateur de vide pour y mesurer la pression,
- le régulateur de vide a une entrée (2) pour l'entrée de vide et une sortie (3) vers le circuit de vide côté patient ainsi qu'un moyen pour réguler la pression appliquée à la sortie du circuit de vide.

6. Système selon la revendication 5,
**caractérisé en ce que**
le régulateur de vide comporte en outre un mécanisme fonctionnant par intermittence avec au moins une connexion mobile (4) entre la position de marche avec communication entre l'entrée de vide (2) et la sortie (3) et une position d'arrêt dans laquelle il n'y a pas de communication entre l'entrée (2) et la sortie (3),
système comportant en outre :
- un corps intermédiaire (5) mobile suivant l'axe longitudinal à l'intérieur du corps externe (1) et ayant une connexion mobile (4) associée de façon que le déplacement axial du corps intermédiaire (5) permette à la connexion (4) d'autoriser ou d'interdire la communication entre l'entrée (2) et la sortie (3) et qui définit une chambre d'extrémité (6) entre le corps externe (1) et l'extrémité transversale du corps intermédiaire (5) pour le déplacement du corps intermédiaire (5) en fonction de la pression atteinte par la chambre (6),
- un piston (7) interne au corps intermédiaire (5) et mobile axialement par rapport au corps intermédiaire (5) de façon que son déplacement autorise ou interdise l'entrée de vide dans la chambre d'extrémité (6) et ainsi le déplacement du corps interne (5).

7. Système selon la revendication 6,
**caractérisé en ce que**
la chambre d'extrémité (6) est soumise à une force élastique.

8. Système selon la revendication 7,
**caractérisé en ce que**
la force élastique est exercée par un ressort (14).

9. Système selon la revendication 6,
**caractérisé en ce que**
le piston (7) comporte des sections d'extrémité (7.1, 7.2) avec des diamètres différents et définissant trois chambres, une première chambre (8) entre l'extrémité de la section la plus petite (7.1) du piston (7), le corps intermédiaire (5) et son extrémité transversale, une seconde chambre (9) définie entre le piston (7) et le corps intermédiaire (5) entre les points de contact dans les deux sections (7.1, 7.2) et finalement une troisième chambre (10) entre le corps intermédiaire (5) et l'extrémité de la section la plus grande (7.2) du piston interne (7).

10. Système selon la revendication 9,
**caractérisé en ce qu'**
il comporte une connexion (11) entre la section mobile (4) et la troisième chambre (10) avec moins de débit que la connexion (4) entre l'entrée (2) et la sortie (3).

11. Système selon la revendication 9,
**caractérisé en ce qu'**
il comporte une connexion (12) entre la chambre d'extrémité (6) et, soit la première chambre (8) soit la seconde chambre (9) selon le déplacement du piston (7).

12. Système selon la revendication 9,
**caractérisé en ce qu'**
il comporte une connexion (13) entre l'entrée (2) et la première chambre (8).

13. Système selon la revendication 6,
**caractérisé en ce que**
le corps intermédiaire (5) se compose de deux corps respectifs.
